(19) Europäisches Patentamt
European Patent Office
Office européen des brevets

(11) Publication number: **0 073 539**
**B1**

(12) # EUROPEAN PATENT SPECIFICATION

(45) Date of publication of patent specification: **15.05.85**

(51) Int. Cl.[4]: **C 07 C 69/63,** C 08 K 5/10

(21) Application number: **82201015.3**

(22) Date of filing: **12.08.82**

(54) **Novel halogenated compounds and fire retardant polymer compositions in which these compounds are incorporated.**

(30) Priority: **29.08.81 NL 8104024**

(43) Date of publication of application:
**09.03.83 Bulletin 83/10**

(45) Publication of the grant of the patent:
**15.05.85 Bulletin 85/20**

(84) Designated Contracting States:
**AT BE DE FR GB IT NL SE**

(56) References cited:
**DE-A-1 543 541**
**DE-A-2 544 513**
**US-A-4 274 998**

(73) Proprietor: **AKZO N.V.**
**Velperweg 76**
**NL-6824 BM Arnhem (NL)**

(72) Inventor: **Hope, Peter**
**16 Lakeside Hollingworth Lake**
**Littleborough (GB)**
Inventor: **Mack, Arthur George**
**22 Hedge Rows Cowm Park**
**Whitworth Rochdale Lancashire (GB)**

(74) Representative: **Sieders, René et al**
**P.O. Box 314**
**NL-6800 AH Arnhem (NL)**

**EP 0 073 539 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to novel compounds of the formula:

$$(Cl_3\ C\ CH_2\ CHY\ \overset{\overset{O}{\|}}{C}O)_p \quad X,$$

where Y is a hydrogen atom or a chlorine atom and p = 1 or 2,

and when p = 1, X = 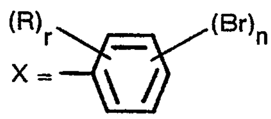 n = 2, 3, 4 or 5

and $n + r \leqq 5$

and when p = 2, X = 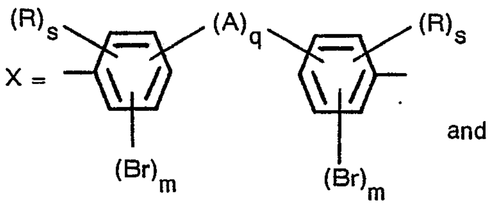 m = 2, 3 or 4

and $m + s \leqq 4$

and q = 0 or 1, where R represents a lower alkyl group containing 1 to 3 carbon atoms, and when q = 1, A has the meaning of a substituted or unsubstituted alkylidene group containing 1 to 3 carbon atoms, $SO_2$, S or O, and to fire retardant polymer compositions in which these compounds are incorporated, and to shaped articles that are entirely or partly made up of such fire retardant polymer compositions.

The novel compounds according to the invention have been found to be particularly suitable for use as fire retardant agent in polymer compositions based on polystyrene and copolymers thereof. Examples of materials which can be fire retarded particularly effectively with the novel compounds of the invention include polyolefins, polyurethanes, unsaturated polyester resins and polyacrylate resins.

In particular, exceptional fire retardant characteristics are imparted by the products of the invention to high impact polystyrene (HIPS) and acrylonitrile butadiene styrene copolymer (ABS), of which polymers it is known that they cannot readily be rendered fire retardant.

Moreover, it has been found that when the fire retardant polymer compositions are processed at elevated temperatures (200° to 250°C), the compounds according to the invention hardly, if at all, give rise to discolouration upon decomposition of the bromine compounds or to corrosion upon contact with metal surfaces.

In this connection it should be added that a structurally related compound, viz. the trichloroacetylchloride derivative of 2,2-bis(3,5-di-bromo-4-hydroxyphenyl)propane is disclosed in Example 6 of German Patent Specification 15 43 541. Though said compound has not explicitly been recommended as a flame-retardant for polymers, some allusion is made to its flame retardant properties if it is to be used as an impregnating agent for wood. In actual practice, however, said compound has been found unsuitable because of its corrosion promoting properties.

Furthermore, British Patent Specification 1 345 211 proposes fire retarding ABS polymers by the incorporation into them of a combination of aromatically bound bromine and aliphatically bound chlorine compounds. In that case the bromine and the chlorine are present in various compounds.

Although this may offer advantages in choosing an optimum chlorine/bromine ratio for a given polymer composition, in actual practice it will be very difficult to realize a perfectly homogeneous distribution of the two compounds in the polymer. As these two compounds will always differ as regards compatibility with the polymer to be rendered fire retardant, eventual inhomogenities must be reckoned with, which manifest themselves in a deterioration of the fire retardant properties of the polymer composition.

Use of the compounds according to the invention has been found to result in an optimum bromine/chlorine ratio without giving problems resulting from a difference in compatibility. Moreover, it has surprisingly been found that for obtaining a particular degree of fire retardancy the present compounds may be incorporated in the polymer compositions in smaller amounts than the known mixtures of chlorine compounds and bromine compounds.

Generally favourable results are obtained when A has the meaning of an isopropylidene group.

Both with respect to the preparation, the favourable compatibility and fire retardant properties it is preferred that use should be made of compounds where in the aromatic ring X has one of the following structures:

X = 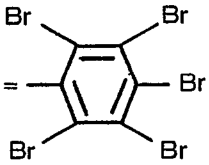 or 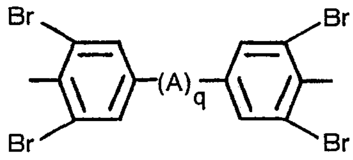

In general, satisfactory fire retardant polymer compositions are obtained by incorporatin therein of 1 to 25% by weight of the novel chlorine- and bromine-containing compounds according to the invention, and 0,5 to 15% by weight of a synergistically active antimony, arsenic, zinc and/or tin compound, said amounts being calculated on the weight of the resulting fire retardant polymer composition.

Examples of suitable antimony, arsenic, zinc and/or tin compounds include antimony chloride, antimony bromide, antimony iodide, antimony oxychloride, arsenic trioxide, arsenic pentoxide, zinc sulphate, zinc oxide, zinc borate and tin (II) oxide. Optimum results are obtained when into these fire retardant polymer compositions the antimony compound $Sb_2O_3$ is incorporated in an amount of 25 to 75, and preferably 33 to 67% by weight, based on the novel compounds of the present invention.

Preparation of the novel compounds according to the invention may be carried out in a manner known from the chemical technology for analogous compounds.

In a preferred procedure 2,4,4,4-tetrachlorobutyl chloride or 4,4,4-trichlorobutyryl chloride is caused to react with a bromated phenol. For best results the reaction is carried out in an inert solution in the presence of a base such as triethylamine or pyridine. Although the temperature of the reaction is not critical, it is preferred that the reaction should be carried out within the range of −10° to 120°C. As the reaction is exothermic, it is preferred to add the acid chloride gradually to the reaction mixture, with cooling, if desired. Heating to higher temperature can be advantageous in certain instances.

The invention will be further described in, but not limited by the following examples.

Example I

Preparation of pentabromophenyl-2,4,4,4-tetrachlorobutyric acid

Pentabromophenol, 49 parts, triethylamine, 10,1 parts, and toluene, 300 parts, were heated until a clear solution was obtained. The solution was cooled to 0°C and 2,4,4,4-tetrachlorobutyryl chloride, 24,5 parts, was slowly run in. Next, the mixture was heated to reflux for 1 hour and triethylamine hydrochloride was removed by filtration. The toluene solution was washed with 100 ml portions of dilute KOH solution and water, dried over anhydrous sodium sulphate and stripped of volatiles, to give a brown oil which slowly crystallized. The crude product was recrystallized from ethanol to give 47 parts of the pentabromophenyl ester of 2,4,4,4-tetrachlorobutyric acid having a melting point of 118°—120°C. This product is hereinafter denoted by the letter A. The same procedure was used for preparing the tribromophenyl ester (product B), and the diesters of

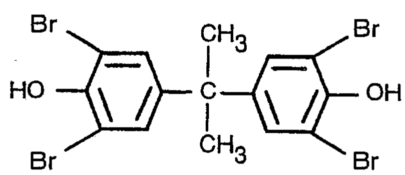 (product C) and of

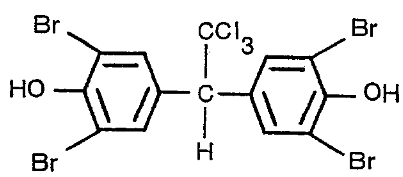 (product D)

Example II

Preparation of bis-trichlorobutyrate ester of tetrabromobisphenol A

136 parts of tetrabromobisphenol A, 39,5 parts of pyridine, 0,6 parts of 4-dimethylaminopyridine and 866 parts of toluene were cooled to 0°C, with stirring, and the system flushed with nitrogen. Subsequently, 105 parts of 4,4,4-trichlorobutyl chloride were added dropwise. When addition was complete the mixture was allowed to cool to room temperature, after which it was stirred for another 5 hours. The pyridine hydrochloride formed was removed by filtration and the toluene filtrate vaporized to give 210 parts of the title compound (compound K), which was a white solid having a melting point of 158°—160°C.

The same procedure was used in preparing the pentabromophenyl ester of trichlorobutyric acid (compound L) having a melting point of 134°—136°C and the tribromophenyl ester of trichlorobutyric acid (compound M) having a melting point of 59°—61°C.

Example III

Two parts of the products of Example I and II were mixed with one part of antimony oxide and, in the amounts indicated below, added to high impact polystyrene.

Mixing with the polystyrene was carried out on a 2-roll mill for 5 minutes at 180°C and thereafter the polymer mix was pressed into a 3,2 m thick plaque at 190°C. Fire retardant performance was evaluated using the UL 94 and BS 2782/508A test methods. The test results obtained are presented in Table I.

TABLE I

| Compound | wt % fire retardant | wt % $Sb_2O_3$ | wt % polystyrene | UL–94 | BS 2782/508A |
|---|---|---|---|---|---|
| A | 10,0<br>9,0 | 5,0<br>4,5 | 85,0<br>86,5 | Vo<br>Vo/$V_1$/F | I<br>I/II |
| B | 11,0 | 5,5 | 83,5 | $V_1$/Vo | I/II |
| | 12,0 | 6,0 | 82,0 | $V_1$/Vo | I |
| C | 12,0<br>11,0 | 6,0<br>5,5 | 82,0<br>83,5 | Vo<br>$V_1$/Vo/F | I<br>I/II |
| D | 10,5 | 5,2 | 84,3 | Vo | I |
| K | 12,5 | 6,8 | 80,7 | Vo | I |
| L | 10,6<br>10 | 6,2<br>5 | 83,2<br>85 | Vo<br>$V_1$ | I<br>I/II |
| M | 12 | 6 | 82 | $V_1$/F | II |

Example IV

In this example the fire retardant effect of the compounds according to the invention are compared with that of a combination of a known bromine-containing compound and a chlorinated compound on the principle of the process of British Patent Specification 1 345 211 discussed in the introductory part of the present application.

The bromine compound used was the 1,2-bis(2,4,6-tribromophenoxy), ethane (compound E) known from British Patent Specification 1 416 815. The aliphatic chlorine-containing compound used was chlorinated PVC. This combination was compared with the compounds A and C according to the invention.

At a weight ratio of 2 parts of fire retardant agent to 1 part of $Sb_2O_3$ the amount of fire retardant material was varied in each experiment until the resulting material satisfied a Vo class I rating, use being made of the UL94 test. The results of the experiments are given in the table below.

TABLE II

| Compound | wt % fire retardant | wt % $Sb_2O_3$ | m moles halogen per 100 g compos. | m moles Br per 100 g compos. |
|---|---|---|---|---|
| E | 14 | 7 | 122 | 122 |
| E + chlorinated PVC | 13 | 6,5 | 130 | 97,5 |
| A | 10 | 5 | 129 | 72 |
| C | 12 | 6 | 150 | 50 |

From the above table it is clear that when use is made of the compounds A and C according to the

invention the bromine content of the polymer compositions that are to be rendered fire retardant can be reduced considerably.

It will be obvious that for reasons of economy it is of great importance that the relatively expensive bromine can be replaced with less expensive chlorine. Also for reasons of environmental protection the use of polymer compositions containing a smallest possible amount of bromine has been found to become increasingly important.

Example V

Samples of the plaques obtained as described in Example III were repressed in a heated compression moulding press, sandwiched between sheets of degreased mild steel at a temperature in the range of 230° to 250°C. After 5 minutes the test specimens were separated from the mild steel and the polymer composition inspected for discolouration on a 1 to 5 scale. The rating 1 indicates that neither the steel nor the polymer are discoloured; the rating 2 is indicative of a dull steel plate surface and a cream coloured polymer; the rating 3 points to slight corrosion of the steel and a pale brown colour of the polymer; the rating 4 means moderate corrosion of the steel plate and a mid brown polymer colour; the rating 5 means heavy corrosion of the steel and a dark brown colour of the polymer. The products A, B, C and D according to the invention were compared with compound E of Example IV and chlorinated PVC.

The results are summarized in the table below.

TABLE III

| Compound | wt % fire retardant | wt % $Sb_2O_3$ | original colour | Polymer discol. | | Corrosion | |
|---|---|---|---|---|---|---|---|
| | | | | 230°C | 250°C | 230°C | 250°C |
| A | 10 | 5 | 1 | 2 | 2–3 | 2 | 2–3 |
| B | 12 | 6 | 1 | 2 | 2 | 1–2 | 2–3 |
| C | 11 | 7 | 1 | 2 | 3 | 2 | 3 |
| D | 10,5 | 5,2 | 1 | 2 | 2–3 | 2 | 2–3 |
| E + chlorinated PVC | 13,0 | 6,5 | 1–2 | – | 4 | – | 5 |

The above table clearly shows the unexpectedly great stability of the compounds according to the invention. This is particularly surprising, considering that in the two compounds the bromine is (aromatically) bonded in the same way.

Example VI

In this example it is demonstrated that the compounds according to the invention are very stable to UV-radiation. In various experiments UV stability was assessed by means of a "Xenotest 150", using accelerated weathering test equipment. Assessments of discolouration were made after periods of 100, 280 and 1000 hours on a 1 to 6 scale, where 1 is a white, 2 a pale yellow, 3 a pale orange, 4 an orange, 5 an orange/brown and 6 a brown polymer. The compounds according to the invention were compared with known compounds such as decabromobiphenyl ether (compound H) and compound E of Example IV.

The results obtained are presented in the table below.

TABLE IV

| Compound | wt % | % $Sb_2O_3$ | original colour | Colour after irradiation for | | |
|---|---|---|---|---|---|---|
| | | | | 100 hours | 280 hours | 1000 hours |
| A | 10 | 5 | 1 | 2–3 | 3–4 | 4–5 |
| B | 12 | 6 | 1 | 2 | 2–3 | 3–4 |
| C | 11 | 7 | 1 | 1–2 | 2 | 3 |
| E | 14 | 7 | 1 | 2 | 2–3 | 5 |
| H | 10 | 5 | 1 | 3 | 4 | 6 |

The above results clearly show that of the compounds according to the invention also the stability to UV-radiation is equal to and in most cases even better than that of the known compounds E and H.

**Claims for the Contracting States: BE DE FR GB IT NL SE**

1. A compound of the formula

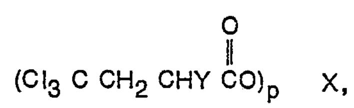

where Y is a hydrogen atom or a chlorine atom and p = 1 or 2,

and when p = 1, X = 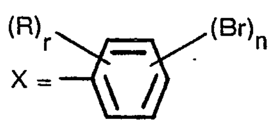 n = 2, 3, 4 or 5 and $n + r \leqq 5$

and when p = 2, X = 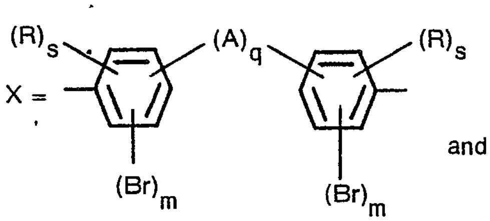 m = 2, 3 or 4 and $m + s \leqq 4$

and q = 0 or 1, where R represents a lower alkyl group containing 1 to 3 carbon atoms, and when q = 1, A has the meaning of a substituted or unsubstituted alkylidene group containing 1 to 3 carbon atoms, $SO_2$, S or O.

2. A compound according to claim 1, characterized in that A has the meaning of an isopropylidene group.

3. A compound according to claim 1, characterized in that

X = 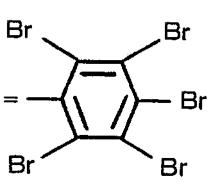 or 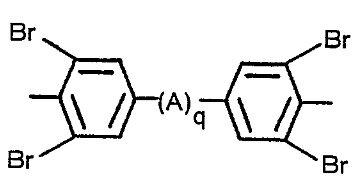

4. A fire retardant polymer composition entirely or partly made up of organic polymers based on polyolefins, polyurethanes, unsaturated polyester resins or polystyrene and copolymers thereof,

characterized in that a fire retardant amount of a compound according to one or more of the preceding claims is incorporated therein.

5. A fire retardant polymer composition according to claim 4, characterized in that 1—25% by weight of a compound according to one or more of the claims 1—3, and 0,5—15% by weight of antimony oxide are incorporated therein, said amounts being based on the weight of the resulting fire retardant composition.

6. A fire retardant polymer composition according to claim 5, characterized in that the antimony oxide is used in an amount of 25 to 75, and preferably 33 to 67% by weight, based on the weight of the compound according to one or more of the claims 1 to 3.

7. Shaped articles entirely or part made up of a fire retardant composition according to one or more of the claims 4 to 6.

**Claims for the Contracting State: AT**

1. A process for the preparation of novel fire retardant compounds, characterized in that a compound is prepared having the formula

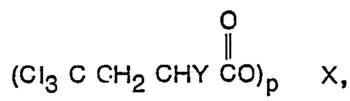

where Y is a hydrogen atom or a chlorine atom and p = 1 or 2,

and when p = 1, X = 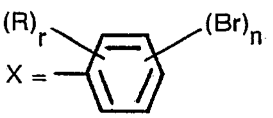 $n = 2, 3, 4$ or $5$ and $n + r \leq 5$

and when p = 2, X = 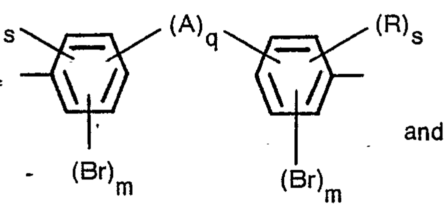 $m = 2, 3$ or $4$ and $m + s \leq 4$

and q = 0 or 1, where R represents a lower alkyl group containing 1 to 3 carbon atoms, and when q = 1, A has the meaning of a substituted or unsubstituted alkylidene group containing 1 to 3 carbon atoms, $SO_2$, S or O.

2. A process for the preparation of a compound according to claim 1, characterized in that a compound having the formula

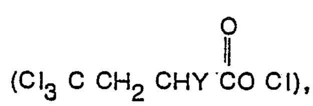

where Y has the meaning indicated in claim 1, is caused to react with a bromated phenol of the formula

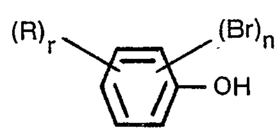 or of,

a bisphenol of the formula

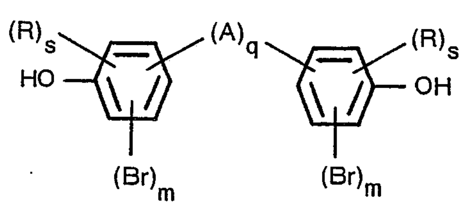

where R, A, r, n,m,s and q have the meaning indicated in claim 1, followed by isolating a compound having the formula as indicated in claim 1.

3. A process according to claim 2, characterized in that the reaction is carried out in an inert solution in the presence of an organic base.

4. A process for rendering a polymer composition entirely or partly made up of organic polymers based on polyolefins, polyurethanes, unsaturated polyester resins or polystyrene and copolymers thereof, fire retardant, characterized in that a fire retardant amount of a compound prepared according to one or more of the preceding claims is incorporated therein.

5. A process according to claim 4, characterized in that 1—25% by weight of a compound prepared according to one or more of the claims 1—3, and 0,5—15% by weight of antimony oxide are incorporated therein, said amounts being based on the weight of the resulting fire retardant composition.

6. A process according to claim 5, characterized in that the antimony oxide is used in an amount of 25 to 75, and preferably 33 to 67% by weight, based on the weight of the compound prepared with a process according to one or more of the claims 1 to 3.

**Patentansprüche für die Vertragsstaaten: BE DE FR GB IT NL SE**

1. Verbindung der Formel

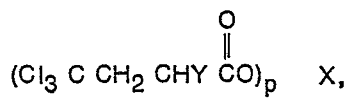

worin Y ein Wasserstoffatom oder ein Choratom ist und p = 1 oder 2, und wenn

p = 1, X = 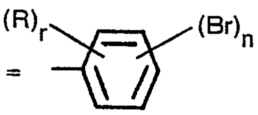 n = 2, 3, 4 oder 5

und $n + r \leq 5$

und wenn

p = 2, X = 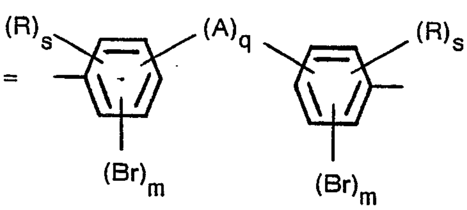 m = 2, 3 oder 4

und $m + s \leq 4$

und q = 0 oder 1, worin R eine Niederalkylgruppe mit 1 bis 3 Kohlenstoffatomen ist, und wenn q = 1, A eine gegebenenfalls substituierte Alkylidengruppe mit 1 bis 3 Kohlenstoffatomen, $SO_2$, S oder O bedeutet.

2. Verbindung nach Anspruch 1, dadurch gekennzeichnet, daß A eine Isopropylidengruppe bedeutet.

3. Verbindung nach Anspruch 1, dadurch gekennzeichnet, daß

X = 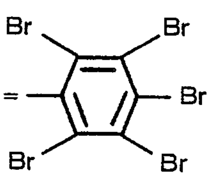 oder 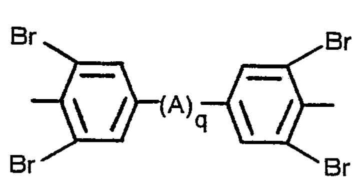

4. Vollständig oder teilweise aus organischen Polymeren auf Basis von Polyolefinen, Polyurethanen, ungesättigten Polyesterharzen oder Polystyrol und Copolymeren hievon hergestellte flammfeste Polymermasse, dadurch gekennzeichnet, daß in sie ein Flammschutzmittel aus einer Verbindung nach einem oder mehreren der vorhergehenden Ansprüche eingearbeitet ist.

5. Flammfeste Polymermasse nach Anspruch 4, dadurch gekennzeichnet, daß in sie 1 bis 25 Gew.-% einer Verbindung nach einem oder mehreren der Ansprüche 1 bis 3 und 0,5 bis 15 Gew.-% Antimonoxid eingearbeitet sind, wobei die Mengen auf das Gewicht der erhaltenen flammfesten Masse bezogen sind.

6. Flammfeste Polymermasse nach Anspruich 5, dadurch gekennzeichnet, daß das Antimonoxid in einer Menge von 25 bis 75 und vorzugsweise 33 bis 67 Gew.-%, bezogen auf das Gewicht der Verbindung nach einem oder mehreren der Ansprüche 1 bis 3, verwendet wird.

7. Vollständig oder teilweise aus einer flammfesten Masse nach einem oder mehreren Ansprüchen 4 bis 6 hergestellte Formkörper.

**Patentansprüche für den Vertragsstaat: AT**

1. Verfahren zur Herstellung von neuen flammfesten Verbindungen, dadurch gekennzeichnet, daß eine Verbindung der Formel

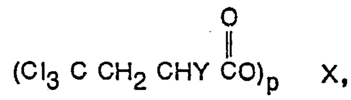

worin Y ein Wasserstoffatom oder ein Chloratom ist und p = 1 oder 2, und wenn

p = 1, X = 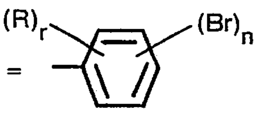 n = 2, 3, 4 oder 5 und $n + r \leqq 5$

und wenn

p = 2, X = 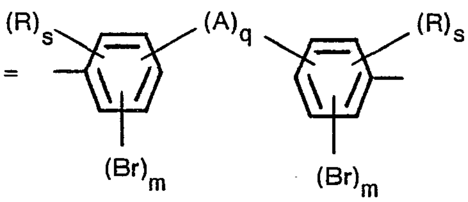 m = 2; 3 oder 4 und $m + s \leqq 4$

und q = 0 oder 1, worin R eine Niederalkylgruppe mit 1 bis 3 Kohlenstoffatomen ist, und wenn q = 1, A eine gegebenenfalls substituierte Alkylidengruppe mit 1 bis 3 Kohlenstoffatomen, $SO_2$, S oder O bedeutet, hergestellt wird.

2. Verfahren zur Herstellung einer Verbindung nach Anspruch 1, dadurch gekennzeichnet, daß eine Verbindung der Formel

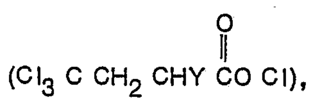

worin Y die in Anspruch 1 angegebene Bedeutung hat, mit einem bromierten Phenol der Formel

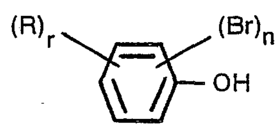

oder einem Bisphenol der Formel

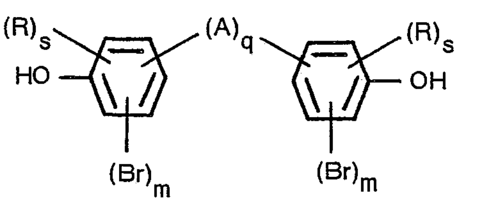

worin R, A, r, n, m, s und q die in Anspruch 1 angegebene Bedeutung haben, reagieren gelassen wird, worauf die Isolierung einer Verbindung der Formel wie in Anspruch 1 angegeben folgt.

3. Verfahren nach Anspruch 2, dadurch gekennzeichnet, daß die Reaktion in einer inerten Lösung in Gegenwart einer organischen Base ausgeführt wird.

4. Verfahren zum Flammfestmachen einer Polymermasse, die vollständig oder teilweise aus organischen Polymeren auf Basis von Polyolefinen, Polyurethanen, ungesättigten Polyesterharzen oder Polystyrol und Copolymeren hievon hergestellt ist, dadurch gekennzeichnet, daß in sie ein Flammschutzmittel aus einer nach einem oder mehreren der vorhergehenden Ansprüche hergestellten Verbindung eingearbeitet wird.

5. Verfahren nach Anspruch 4, dadurch gekennzeichnet, daß 1 bis 25 Gew.-% einer nach einem oder mehreren der Ansprüche 1 bis 3 hergestellten Verbindung und 0,5 bis 15 Gew.-% Antimonoxid

eingearbeitet werden, wobei die Mengen auf das Gewicht der erhaltenen flammfesten Masse bezogen sind.

6. Verfahren nach Anspruch 5, dadurch gekennzeichnet, daß das Antimonoxid in einer Menge von 25 bis 75 und vorzugsweise 33 bis 67 Gew.-% bezogen auf das Gewicht der nach einem Verfahren gemäß einem oder mehreren der Ansprüche 1 bis 3 hergestellten Verbindung, verwendet wird.

**Revendications pour les Etats contractants: BE DE FR GB IT NL SE**

1. Un composé de formule

$$(Cl_3\ C\ CH_2\ CHY\ \overset{\overset{O}{\|}}{C}O)_p\quad X,$$

dans laquelle Y est un atome d'hydrogène ou un atome de chlore et p = 1 ou 2,

et, lorsque p = 1, X = 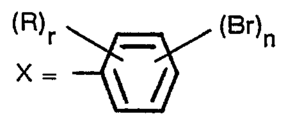 $n = 2, 3, 4$ ou 5 et $n + r \leqslant 5$

et, lorsque p = 2, X = 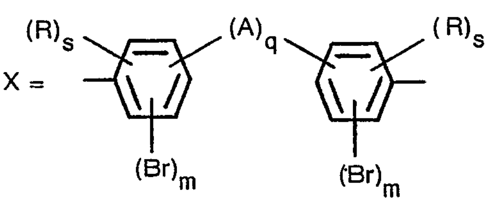

$m = 2, 3$ ou 4 et $m + s \leqslant 4$ et $q = 0$ ou 1, dans laquelle R représente un groupe alkyle inférieur contenant 1 à 3 atomes de carbone et, lorsque q = 1, A désigne un groupe alkylidène substitué ou non substitué contenant 1 à 3 atomes de carbone, $SO_2$, S ou O.

2. Un compose' selon la revendication 1, caractérisé en ce que A désigne un groupe isopropylidène.

3. Un composé selon la revendication 1, caractérisé en ce que

X = 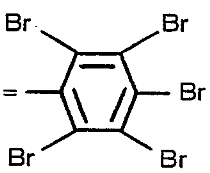 ou 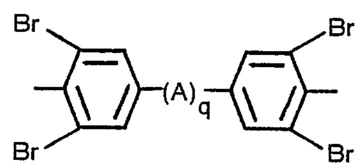

4. Une composition polymère ignifugeante entièrement ou partiellement faite de polymères organiques à base de polyoléfines, de polyuréthannes, de résines polyester insaturées ou de polystyrène et de ses copolymères, caractérisée en ce que l'on y incorpore une quantité ignifugeante d'un composé selon l'une ou plusieurs des revendications précédentes.

5. Une composition polymère ignifugeante selon la revendication 4, caractérisée en ce que l'on y incorpore 1—25% en poids d'un composé selon l'une ou plusieurs des revendications 1—3 et 0,5—15% en poids d'oxyde d'antimoine, les dites quantités étant basées sur le poids de la composition ignifugeante résultante.

6. Une composition polymère ignifugeante selon la revendication 5, caractérisée en ce que l'oxyde d'antimoine est employé en quantité de 25 à 75, et de préférence de 33 à 67%, % en poids, par rapport au poids du composé selon l'une ou plusieurs des revendications 1 à 3.

7. Articles profilés entièrement ou partiellement faits de la composition ignifugeante selon l'une ou plusieurs des revendications 4 à 6.

**Revendications pour l'Etat contractant: AT**

1. Un procédé de préparation de nouveaux composés ignifugeants, caractérisé en ce que l'on prépare un composé de formule

$$(Cl_3\ C\ CH_2\ CHY\ \overset{\overset{O}{\|}}{C}O)_p\quad X,$$

dans laquelle Y est un atome d'hydrogène ou un atome de chlore et p = 1 ou 2,

et, lorsque p = 1, X = 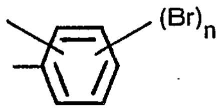 n = 2, 3, 4 ou 5

et $n + r \leqslant 5$

et, lorsque p = 2, X = 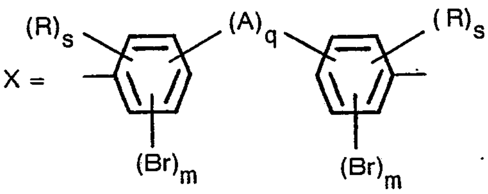

m = 2, 3 ou 4 et $m + s \leqslant 4$ et q = 0 ou 1, dans laquelle R représente un groupe alkyle inférieur contenant 1 à 3 atomes de carbone et, lorsque q = 1, A désigne un groupe alkylidène substitué ou non substitué contenant 1 à 3 atomes de carbone, $SO_2$, S ou O.

2. Un procédé de préparation d'un composé selon la revendication 1, caractérisé en ce que l'on fait réagir un composé de formule

$$(Cl_3\,C\,CH_2\,CHY\,\overset{O}{\overset{\|}{C}}O\,Cl),$$

, dans laquelle Y a la signification indiquée dans la revendication 1, avec un phénol bromé de formule

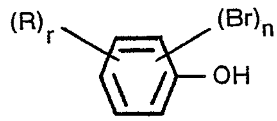 ou avec

un bisphénol de formule

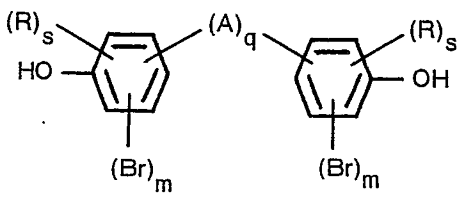

dans laquelle R, A, r, n, m, s et q sont les significations indiquées dans la revendication 1, puis on isole un composé de formule indiquée dans la revendication 1.

3. Un procédé selon la revendication 2, caractérisé en ce que la réaction est réalisée en solution inerte en présence d'une base organique.

4. Un procédé pour rendre ignifugeante une composition polymère entièrement ou partiellement faite de polymères organiques à base d'oléfines, de polyuréthannes, de résines polyester insaturées ou de polystyrène et de ses copolymères, caractérisé en ce que l'on y incorpore une quantité ignifugeante d'un composé préparé selon l'une ou plusieurs des revendications précédentes.

5. Un procédé selon la revendication 4, caractérisé en ce que l'on y incorpore 1—25% en poids d'un composé préparé selon l'une ou plusieurs des revendications 1—3 et 0,5—15% en poids d'oxyde d'antimoine, les dites quantités étant basées sur le poids de la composition ignifugeante résultante.

6. Un procédé selon la revendication 5, caractérisé en ce que l'on emploie l'oxyde d'antimoine en quantité de 25 à 75, et de préférence de 33 à 67,% en poids, par rapport au poids du composé préparé par un procédé selon l'une ou plusieurs des revendications 1 à 3.